# EUROPEAN PATENT APPLICATION

(11) **EP 2 295 420 A1**
(43) Date of publication of application: **16.03.2011**
(21) Application number: 09290701.3
(22) Date of filing: 11.09.2009
(51) Int. Cl.: C07D 307/52, C07F 15/04, C08F 112/08, C08F 4/602

(54) **Carbonylamino fulvene compounds and their use as ligands of nickel based catalysts for the production of isotactic polystyrene**

(71) Applicant: Total Petrochemicals France, 92047 Paris La Défense Cedex (FR); CENTRE NATIONAL DE LA RECHERCHE SCIENTIFIQUE (CNRS), 75016 Paris (FR)
(72) Inventor: Lansalot-Matras, Clément, 35132 Vezin-le-coquet (FR); Lavastre, Olivier, 35490 Gahard (FR); Razavi, Abbas, 7000 Mons (BE); Vuillemin, Bruno, 64230 Lescar (FR); Duc, Michel, F-64000 Pau (FR)
(74) Representative: Neel, Henry

(57) **Abstract**

In one aspect the invention features 1-(4-ter-butylbenzoyl-6-(1-(furan-2-methyl)amino)-6-(4-terbutylphenyl) fulvene (I) and a process for prearing it, wherein 1-4-tertbutylbenzoyl-6-hydroxy-6-4-tertbutylphenylfulvene is reacted with furfurylamine.
Another subject-matter of the invention is a nickel acetate (Ni(OAc)₂) - ligand complex, wherein the ligand is the compound as defined above, and a process for producing this complex, wherein a compound as defined above is reacted with Ni(OAc)₂.
Another subject-matter of the invention is a process for preparing isotactic polystyrene using the above complex having reacted with an alumoxane as a catalyst system, and isotactic polystyrene obtainable by this process.

## Description

The present invention relates to new carbonylamino fulvene compounds, to the use of such new compounds as ligands of ligand-nickel based catalysts for the production of isotactic polystyrene, to the production of such catalysts, and to the production of isotactic polystyrene using such catalysts.

Isotactic polystyrene was discovered half a century ago and was first produced with heterogeneous catalysts as described for example by Natta *et al*. **[1]**, **[2]** or by Overberger *et al*. **[3],** or by Kern *et al*. **[4].**

The isotactic polystyrene was also produced via anionic polymerization as described by Cazzaniga *et al*. **[5]** or by Makino *et al.* **[6].**

There are few examples of true single-site catalysts that form isotactic polystyrene. Among those is the titanium based system supported by a 1,4-dithiabutane-bridged bis(phenolato) [OSSO] described by Okuda *et al*. **[7], [8], [9].** Ansa-bridged bis(indenyl) allyl yttrium and neodium complexes recently described for example **[10], [11]** by Carpentier *et al*. also formed very pure IPS.

Although a range of homogeneous catalysts based on metals others than those of groups 3 and 4 have been reported to give isotactic-enriched polystyrene for example by Gomes *et al*. **[12]** or by Porri *et al*. **[13]** or by Dias *et al.* **[14]**, or by Po *et al*. **[15]**, there is no example of true single-site catalyst based on nickel that produces isotactic polystyrene.
[1] G. Natta, P. Pino, P. Corradini, F. Danusso, E. Mantica, G. Mazzanti, G. Moraglio, J. Am. Chem. Soc. 1955, 77, 1708;
[2] G. Natta, P. Corradini, Makromol. Chem. 1955, 16, 77
[3] C. Overberger, H. Mark, J. Polym. Sci. 1959, 35, 381
[4] R. J. Kern, H. G. Hurst, W. J. Richard, J. Polym. Sci. 1960, 45, 195
[5] L. Cazzaniga, R. E. Cohen, Macromolecules 1989, 22, 4125
[6] T. Makino, T. E. Hogen-Esch, Macromolecules 1999, 32, 5712
[7] C. Capacchione, A. Proto, H. Ebeling, R. Mulhaupt, K. Moller, T. P. Spaniol, J. Okuda, J. Am. Chem. Soc. 2003, 125, 4964
[8] K. Beckerle, R. Manivannan, B. Lian, G.-J. M. Meppelder, G. Raabe, T. P. Spaniol, H. Ebeling, F. Pelascini, R. Mülhaupt, J. Okuda, Angew. Chem. 2007, 119, 4874
[9] Angew. Chem. Int. Ed. 2007, 46, 4790
[10] A.-S. Rodrigues, E. Kirillov, T. Roisnel, A. Razavi, B. Vuillemin, J.-F. Carpentier Angew. Chem. 2007, 119, 7378-7381
[11] A.-S. Rodrigues, E. Kirillov, A. Razavi, J.-F. Carpentier EP1818337A1, 2006
[12] R. Ascenso, A. R. Dias, P. T. Gomes, C. C. Romao, I. Tkatchenko, A. Revillon, Q. T. Pham, Macromolecules 1996, 29, 4172
[13] G. L. Crossetti, C. Bormioli, A. Ripa, A. Giarrusso, L. Porri, Macromol. Rapid Commun. 1997, 18, 801
[14] M. L. Dias, G. L. Crossetti, C. Bormioli, A. Giarusso, L. C. de Santa Maria, F. M. B. Coutinho, L. Porri, Polym. Bull. 1998, 40, 689
[15] R. Po, N. Cardi, R. Santi, A. M. Romano, C. Zannoni, S. Spera, J. Polym. Sci. Part A 1998, 36, 2119

The present invention mitigates this drawback and describes a new carbonylaminofulvene nickel acetate complex that is highly efficient after activation, for example with methylaluminoxane (MAO), for the isospecific homopolymerization of styrene.

In one aspect the invention features nickel acetate catalyst complexed with 1-(4-ter-butylbenzoyl)-6-(1-(furan-2-methyl)amino)-6-(4-tertbutylphenyl) fulvene of formula (I):

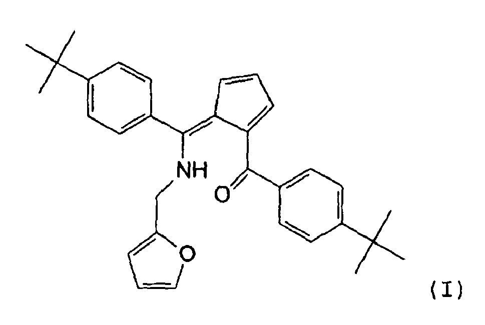

It was also interestingly found that the compound of formula (I) was the sole effective ligand leading to the high-yield production of highly isotactic polystyrene in the presence of a nickel acetate catalyst, among all the aminofulvene compounds characterised by the generic chemical formula (II) 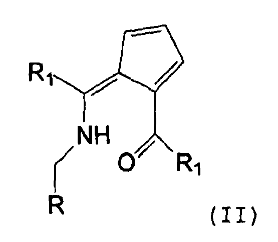 wherein:
- R is: 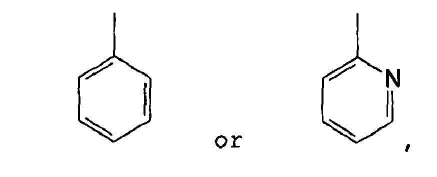 and
- R₁ is: 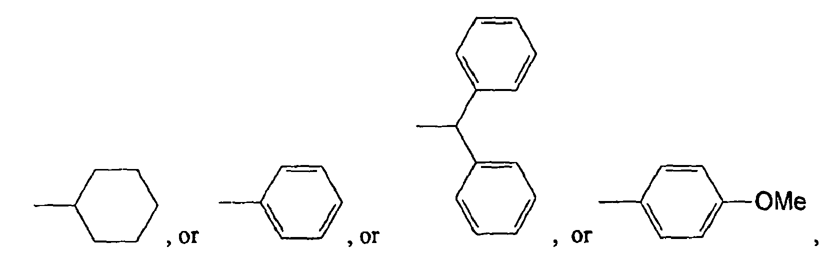 or

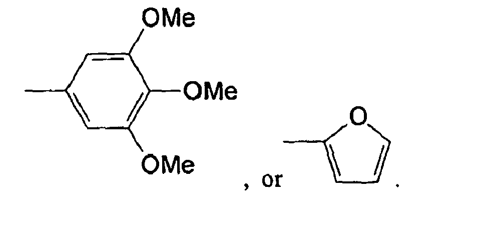

The compound (I) was defined by its major tautomer form, namely the carbonylamino form. It is considered that the following form of the compound (I) can also exist:

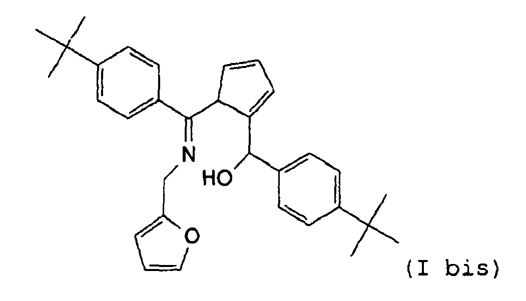

Another subject-matter of the invention is a process for preparing the compound (I) as defined above, wherein the compound of formula (III), namely 1-4-tertbutylbenzoyl-6-hydroxy-6-4-tertbutylphenylfulvene: 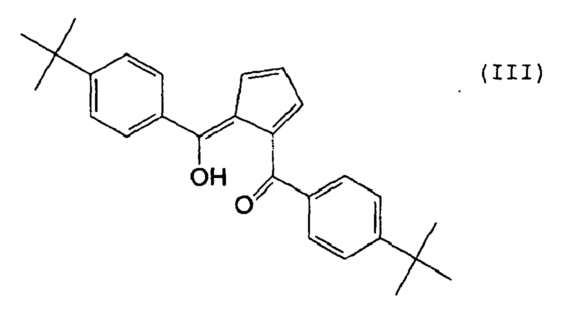 is reacted with the amine of formula (IV), namely furfurylamine:

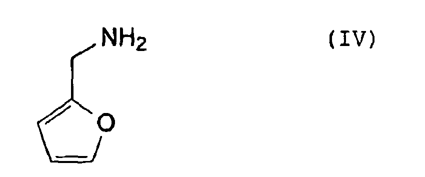

Preferably, the reaction is carried out with a molar ratio of (III):(IV) from 1:1.1 to 1:2.0.

Preferably, the reaction is carried out at a temperature of about 80 to about 110 °C, during about 8 to about 24 hours, in a solvent and optionally in the presence of a catalytic amount of an acid. Advantageously the solvent is an alcohol, e.g., ethanol. The acid is advantageously a sulfonic acid, e.g., paratoluene sulfonic acid. Advantageously the reaction is carried out in ethanol used as the solvent medium and in the presence of a catalytic amount of paratoluene sulfonic acid.

Another subject-matter of the invention is a Ni(OAc)₂ - ligand complex, wherein the ligand is the compound as defined above.

Another subject-matter of the invention is a process for producing the complex as defined above, wherein a compound as defined above is reacted with Ni(OAc)₂ ideally at room temperature (typically around 20-25°C) , during about 1 to about 2 hours, in a solvent medium, and the complex is retrieved by removing the solvent. The solvent is, e.g., tetrahydrofuran (THF).

Another subject-matter of the invention is an active catalyst system, for the isospecific homopolymerisation of styrene that consists of the complex of claim 5 having reacted with an alumoxane. The preferred alumoxanes comprise oligomeric linear and/or cyclic alkyl alumoxanes represented by the formula :
R-(Al-O)ₙ-AlR₂ for oligomeric, linear alumoxanes
and
(-Al-O-)ₘ for oligomeric, cyclic alumoxanes,
wherein n is 1-40, preferably 10-20, m is 3-40, preferably 3-20 and R is a C₁-C₈ alkyl group and preferably methyl. Methylalumoxane (MAO) is preferably used.

Another subject-matter of the invention is a process for preparing isotactic polystyrene, comprising the steps of:
(a) injecting the active catalyst system of claim 7 into a reactor;
(b) injecting styrene into said reactor;
(c) maintaining under polymerisation conditions;
(d) retrieving isotactic polystyrene.

Another subject-matter of the invention is an isotactic polystyrene having an isotactic index of at least about 93% obtainable by the process of claim 8.

Preferably, the polydispersity index Ip of the isotactic polystyrene is from about 1 to about 15.

Preferably, the polystyrene molecular-weight distribution is essentially monomodal.

Preferably, the number average molecular weight Mn of the isotactic polystyrene is from about 30,000 to about 100,000 g/mol.

Preferably, the isotactic index of the isotactic polystyrene is at least about 95%.

The following examples illustrate the present invention, without limiting its scope. In these examples, the following abbreviations have been used:
- IPS: isotactic polystyrene
- MAO: methylaluminoxane
- PS: polystyrene
- PTSA: paratoluene-sulfonic acid
- THF: tetrahydrofuran
- DSC: differential scanning calorimetry
- GPC: gel permeation chromatography
- HRMS: high resolution mass spectrography
- NMR: nuclear magnetic resonance
- SEC: size exclusion chromatography

### List of figures

- FIGURE 1 represents the molecular structure of ligand 2 (Example 1) obtained by X-ray.
- FIGURE 2 represents the GPC trace of IPS (Table 1, Example 4)
- FIGURE 3 (Graphs 1 and 2): Semi-logarithmic plots as a function of time, molecular weight and polydispersity index obtained by GPC from aliquots withdrawn during the polymerization as a function of styrene conversion. Conditions [Ni(OAc)/21/MAO/styrene=1/20/1000, 30 °C, styrene:toluene=1:2 in vol.

### Examples

Chemicals were purchased from commercially available sources and used without purification and the solvents were purified following standard procedures.

Mass spectra were obtained with a high resolution mass spectrometer Varian MAT 311 and microanalyses were carried out on a Flash EA1112 CHNS/O Thermo Electron (Centre Régional de Mesures des Physiques de l'Ouest, Rennes, France).

Crystallographic data collection, unit cell constant and space group determination were carried out on an automatic 'Enraf Nonius FR590' NONIUS Kappa CCD diffractometer with graphite monochromatized Mo-Kα radiation at 120 K. The cell parameters were obtained with Denzo and Scalepack with 10 frames (psi rotation: 1° per frame). The structure was solved with SIR-97. The whole structure was refined with SHELXL97 by the full-matrix least-square techniques.

Molecular weights and molecular weight distributions of the polymers were determined by SEC at 40 °C in chloroform. A Dionex P680 HPLC pump equipped with the PL-ELS 1000 from Polymer Laboratories was used and connected to a PLgel 10 µm MIXED-B column. PS standards were used for calibration (∼1 mg/mL; injection volume 20 µL; flow rate 1 mL/min).

Differential scanning calorimetry analysis was performed on a TA-Instrument DSC2010.

The NMR spectra were recorded either on a Brücker ARX 200 spectrometer, at 200 MHz for ¹H spectra and at 50 MHz for ¹³C spectra, or on a Brücker AC 300P at 300 MHz for ¹H spectra and at 75 MHz for ¹³C spectra. Chemical shifts are reported in δ (ppm) using CDCl₃ as the reference solvent unless otherwise stated.

The triads tacticity of the PS was determined by using the area ratios of the splitting ipso carbon in the ¹³C NMR spectra recorded at 300 MHz as already described for example by Ishihara *et al.* N. Ishihara, T. Seimiya, M. Kuramoto, and M. Uoi Macromolecules 1986, 19, 2464-2465

### Example 1: Synthesis of 1-(4-tertbutrylbenzoyl)-6-(1-(furan-2-methyl)amino)-6-(4-tertbutylphenyl) fulvene 2

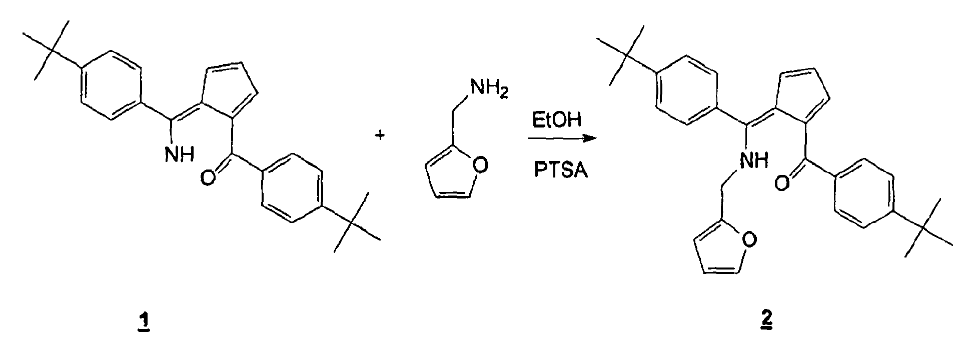

Carbonylaminofulvene ligand **2** was synthesised following the methods similar to those described for example in Lloyd and Preston D. Lloyd, N.W. Preston, J. Chem. Soc. C 1969, 2464-2469

In a Schlenk tube under argon, 1-4-tertbutylbenzoyl-6-hydroxy-6-4-tertbutylphenylfulvene **1** (342 mg / 0.885 mmol) were introduced with furfurylamine (156 µl / 1.77 mmol) and 1 mg of paratoluene-sulfonic acid (PTSA) and 10 ml of ethanol.

The mixture was heated for 8 h at 90 °C. Ligand **2** was recristallised from ethanol to yield a yellow solid (327 mg / 0.702 mmol / 79 %).
**¹H NMR** (CDCl₃, 300 MHz, ppm) δ: 14.14 (1H, s, N²⁰**H**), 7.56 (2H, d, *J*=0.03 Hz, C¹⁵**H**), 7.42-7.27 (6H, m, C⁸**H**, C⁹**H**, C¹⁶**H**), 7.23 (1H, s, C²**H**), 7.03 (1H, d, *J*=0.01 Hz, C²⁵**H**), 6.32 (1H, t, *J*=0.01 Hz, C²⁴**H**), 6.18-6.09 (1H, m, C³**H**, C⁴**H** and C²³**H**), 4.32 (2H, d, *J*=0.02 Hz, C²¹**H**), 1.26 (18H, d, *J*=0.03 Hz, C¹²**H**₃ and C¹⁹**H**₃)
**¹³C NMR** (CDCl₃, 50 MHz, ppm) δ: 191.09 (C¹³), 166.41 (C⁶), 153.17 (C¹⁷) 152.91 (C¹⁰), 149.98 (C²²), 142.66 (C²⁵), 139.62 (C¹⁴), 139.46 (C⁷), 135.72 (C²), 130.88 (C⁴), 128.93 (C⁸), 128.38 (C⁹), 125.39 (C¹⁶), 125.15 (C¹⁵), 124.71 (C¹), 119.93 (C⁵), 117.92 (C³), 110.58 (C²⁴), 108.08 (C²³), 42.99 (C²¹) 34.88 (C¹¹ and C¹⁸), 31.39 (C¹² and C¹⁹).
**HRMS:** Calculated. for M+. (C₃₂H₃₅NO₂) m/z= 465.26678, found 465.2669 (0 ppm).
**Anal.** Calculated. for C₃₂H₃₅NO₂: C: 82.54, H: 7.58, N: 3.01, O: 3.01
   found C: 81.98, H: 7.57, N: 3.14.
**Cristallography**. Single crystals of compound **2** suitable for a single crystal Xray determination were obtained by evaporation of a saturated solution in THF.

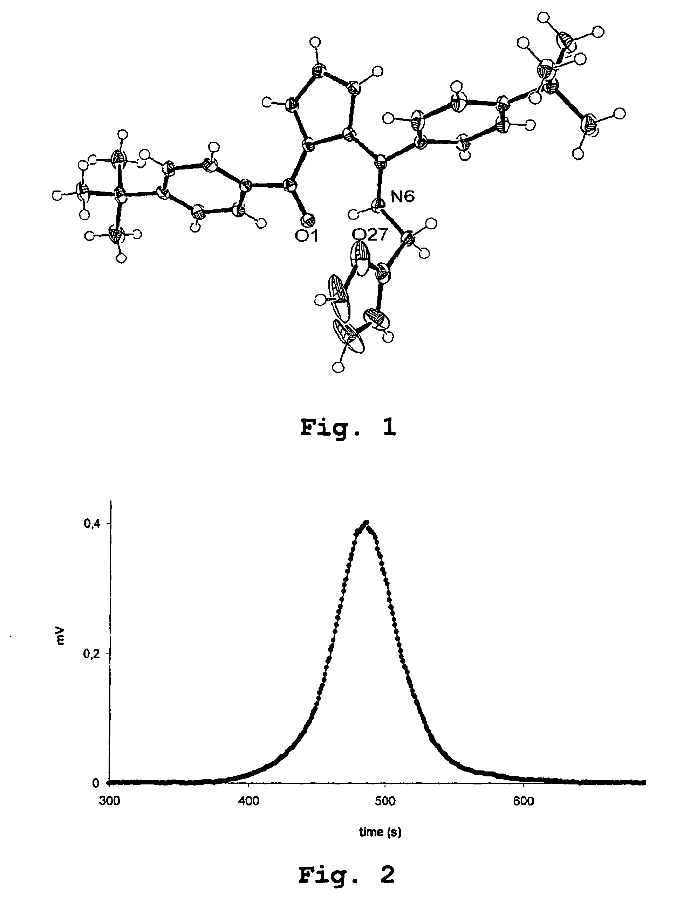

The molecular structure of **2** is shown in Figure 1. Empirical formula: C₃₂H₃₅NO₂, Formula weight: 465.61, Temperature: 100(2) K, Wavelength: 0.71073 Å, Crystal system: Monoclinic, Space group: P 21/c, Unit cell dimensions: a=6.2266(11) Å, b=18.665(4) Å, c=23.267(4) Å, alpha=90°, beta=93.008(10)°, gamma=90°, Volume: 2700.4(9) Å³, Z=4, Calculated density: 1.145 mg/m³, Absorption coefficient: 0.070 mm⁻¹, F(000): 1000, Crystal size: 0.6 x 0.12 x 0.1 mm, Theta range for data collection: 3.28 to 27.44°, Limiting indices: -7 ≤ h ≤ 8, -24 ≤ k ≤ 24, -29 ≤ 1 ≤ 29, Reflections collected / unique: 25919 / 6085 [R(int) = 0.0529], Completeness to theta: 27.44 (99.0 %), Absorption correction: Semi-empirical from equivalents, Max. and min. transmissions: 0.993 and 0.952, Refinement method: Full-matrix least-squares on F², Data / restraints / parameters: 6085 / 0 / 326, Goodness-of-fit on F²: 1.016, Final R indices: [I>2sigma(I)] R1=0.0464, wR2=0.1002, R indices (all data): R1=0.0775, wR2=0.1138, Largest diff. peak and hole: 0.315 and -0.247 e. Å⁻³.

### Example 2: Synthesis of Ni(OAc)₂ / ligand 2 complex.

2.16 mg (8.7 µmol) of Ni(OAc)₂ were dried under vaccuum during 1 night at 100 °C, and introduced in a Schlenk tube with 8.10 mg (17.4 mmol) of ligand **2** and 100 µL of THF.

The mixture was placed under stirring for 2 h at room temperature.

The solvent was evaporated under vacuum overnight to yield a yellow solid. The complex was used without further purification for the polymerisation tests as the metallic catalyst component.

### Examples 3 to 12:

Typical procedure for the polymerisation of styrene.

The metallic catalyst component (8.7 umol) was activated with 20 equivalents (37 µL) of MAO. The solution was stirred for 30 minutes at room temperature and then diluted with 2 mL of toluene. The styrene (1 mL, 8.7 mmol) was then added and the mixture was stirred for a period of time of 1 hours at 30 °C otherwise mentioned. Finally, the polymerisation experiment was stopped by pouring the content of the Schlenk tube in a large excess of methanol containing a small amount of hydrochloric acid. The coagulated polymer was washed with methanol, filtered and finally dried under vacuum. The results are summarised in Table I.

GPC traces of the polymers were all monomodal as shown in Figure 2, which is consistent with a single site behaviour.

The microstructure of the PS was determined by ¹³CNMR spectroscopy and revealed a high content of IPS (>90 %) which was confirmed by a melting temperature (Tm) measured at 222 °C (determined by DSC). A typical ¹³C NMR spectrum of IPS obtained according to Example 4 and presents a single sharp resonance at δ=146,5 ppm for the phenyl ipso carbon.

High content of IPS were also obtained at higher and lower temperature (Examples 3, 4 and 5), without solvent (Example 6), and at lower catalyst concentration (Examples 7 and 8). An excess of MAO (100 equivalents) resulted in a decrease of the amount of IPS (51 %).

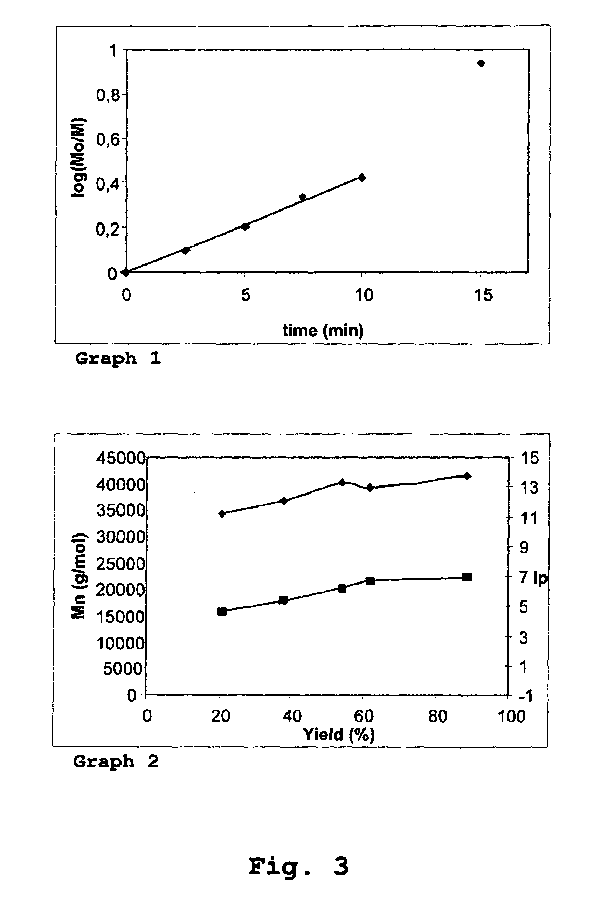

A kinetic study was also carried out with this catalytic system. Semi-logarithmic plots of the conversion of styrene as a function of time, and evolutions of number average molecular weight (Mn) and polydispersity index (Ip) as a function of styrene conversion are plotted in the Graph. 1 and 2 of Figure 3. A linear regime is observed during the first 60 % of styrene conversion which indicates a) a first-order styrene conversion and b) the concentration of the active centers was constant during the first 60 % of the conversion, which is in good agreement with the quasi steady-state approximation. Mn and Ip slightly increase with the styrene conversion, respectively Mn from 34 000 - 41 000 g/mol, and Ip from 4,7 to 7,0. Such characteristics indicate that there is no control of molecular weight.

**TABLE I**

| **Ex** | **Ni/L (mol/mol)** | **Ni/MAO/styrone (mol/mol/mol)** | **Solvent** | **Time (min)** | **T (°C)** | **Yield (%)** | **Mp (g/mol)** | **Mw (g/mol)** | **Mn (g/mol)** | **Ip** | **Tacticity (%)** | | | **Tm (°C)** |
|---|---|---|---|---|---|---|---|---|---|---|---|---|---|---|
| | | | | | | | | | | | **rr** | **mr** | **mm** | |
| 3 | 1/2 | 1/20/1000 | toluene | 60 | 0 | 26 | 79 160 | 511 715 | 38 955 | 13.14 | 2 | 8 | 90 | n.m. |
| 4 | 1/2 | 1/20/1000 | toluene | 60 | 30 | 93 | 81 910 | 223 700 | 30 780 | 7.27 | 2 | 7 | 91 | 222 |
| 5 | 1/2 | 1/20/1000 | toluene | 60 | 90 | 42 | 19 790 | 54 860 | 7 270 | 7.54 | 1 | 10 | 89 | 207 |
| 6 | 1/2 | 1/20/1000 | - | 60 | 30 | 64 | 94 120 | 363 130 | 38 823 | 9.35 | 1 | 8 | 91 | 213 |
| 7 | 1/2 | 1//20/2000 | toluene | 60 | 30 | 35 | 129 010 | 474 760 | 33 240 | 14.28 | 2 | 8 | 90 | 214 |
| 8 | 1/2 | 1/20/3000 | toluene | 60 | 30 | 40 | 119 460 | 464 830 | 29 600 | 15.70 | 1 | 7 | 92 | 214 |
| 9 | 1/2 | 1/100/1000 | toluene | 60 | 30 | 76 | 36 340 | 35 762 | 18 070 | 1.98 | 18 | 31 | 51 | - |
| 10 | 1/1 | 1/20/1000 | toluene | 10 | 30 | 40 | 56 940 | 110 260 | 27 670 | 3.98 | 4 | 14 | 82 | n.m. |
| 11 | 1/2 | 1/20/1000 | toluene | 10 | 30 | 39 | 66 530 | 175 975 | 28 340 | 6.21 | 1 | 10 | 89 | n.m. |
| 12 | 1/3 | 1/20/1000 | toluene | 10 | 30 | 14 | 111 860 | 361 183 | 45 920 | 7.87 | 1 | 10 | 89 | n.m. |

| | | | | | | | | | | | | | | |
|---|---|---|---|---|---|---|---|---|---|---|---|---|---|---|
| n.m. not measured | | | | | | | | | | | | | | |

### Examples 13 and 14: Synthesis of compounds of formulae 4 and 5 and of each corresponding Ni(OAc)₂ / ligand complex

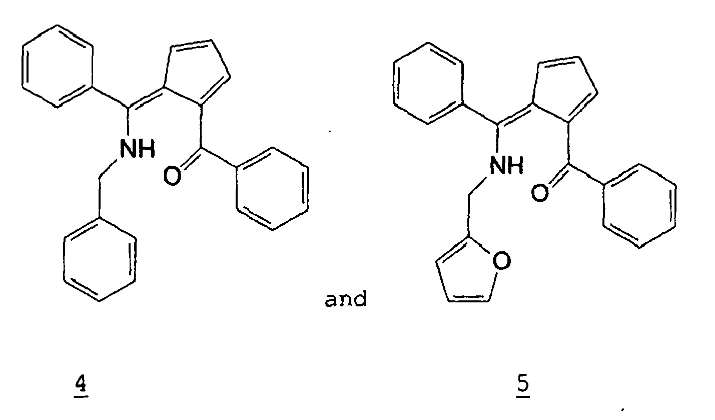

The synthesis of compounds 4 and 5 was conducted as in Example 1, except that the amine was replaced by benzylamine and by furan-2-yl methylamine in order to obtain the ligands of the formulae 4 and 5, respectively.

Then Ni(OAc)₂/ligand 4 and Ni(OAc)₂/ligand 5 were prepared as in Example 2.

### Example 15 to 17 : Polymerisation of styrene

The polymerisation of styrene was conducted as in Examples 3 to 12 with the activated Ni(OAc)₂/ligand 3 catalyst (Example 15), the activated Ni(OAc)₂/ligand 4 catalyst (Example 16) and with the activated Ni(OAc)₂ catalyst (Reference Example 15)

Results are indicated in the following Table II.

**Table II:**

| **Ex** | **Ni/L (mol/mol)** | **Ni/MAO/styrene (mol/mol/mol)** | **Yield (%)** | **Mp (g/mol)** | **Mn (g/mol)** | **Ip** | **Tacticity (%)** | | |
|---|---|---|---|---|---|---|---|---|---|
| | | | | | | | **rr** | **mr** | **mm** |
| 13 | 1/2 | 1/20/1000 | 72 | 75 970 | 41 409 | 2.82 | 21 | 23 | 56 |
| 14 | 1/2 | 1/20/1000 | 96 | 66 860 | 50 231 | 1.91 | 18 | 25 | 57 |
| 15 | 1/2 | 1/20/1000 | 53 | 47 500 | 22 200 | 1.81 | 19 | 28 | 52 |

| | | | | | | | | | |
|---|---|---|---|---|---|---|---|---|---|
| T = 30°C, 1h, toluène. | | | | | | | | | |

## Claims

1. 1-(4-ter-butylbenzoyl)-6-(1-(furan-2-methyl)amino)-6-(4-tertbutylphenyl) fulvene and its tautomeric form, as described by formulae (I) and (I bis), respectively 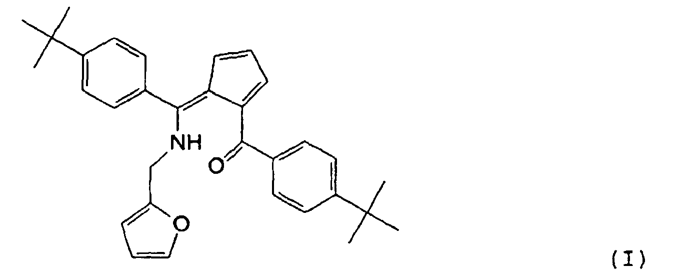 and

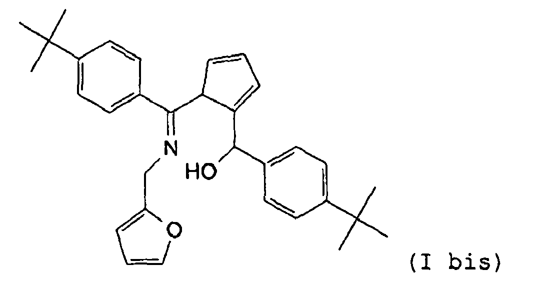

2. Process for preparing the compounds of claim 1, wherein the compound of formula (III), namely 1-4-tertbutylbenzoyl-6-hydroxy-6-4-tertbutylphenylfulvene: 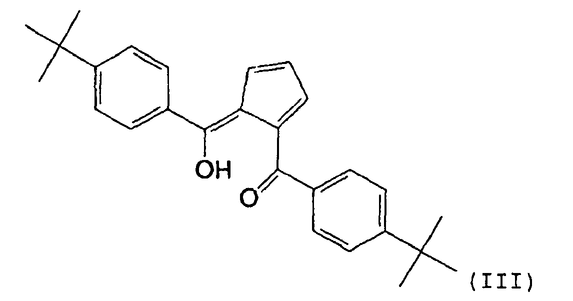 is reacted with the amine of formula (IV), namely furfurylamine:

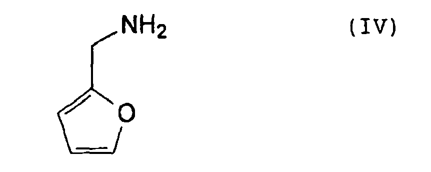

3. Process according to claim 2, wherein the reaction is carried out with a molar ratio of (III):(IV) from 1:1.1 to 1:2.0.

4. Process according to anyone of claims 3 and 4, wherein the reaction is carried out at a temperature of about 80 to about 110°C, during about 8 to about 24 hours, in a solvent and optionally in the presence of a catalytic amount of an acid.

5. Ni(OAc)₂ - ligand complex, wherein the ligand is the compound as defined in claim 1.

6. Process for producing the complex as defined in claim 5, wherein the compound as defined in claim 1 is reacted with Ni(OAc)₂ at ambient temperature, i.e., around 20-25°C, during about 1 to about 2 hours, in a solvent medium, and the complex is retrieved by removing the solvent.

7. An active catalyst system, for the isospecific homopolymerisation of styrene that consists of the complex of claim 5 having reacted with an alumoxane, methylaluminoxane (MAO) being most preferred.

8. Process for preparing isotactic polystyrene, comprising the steps of:
(a) injecting the active catalyst system of claim 7 into a reactor;
(b) injecting styrene into said reactor;
(c) maintaining under polymerisation conditions;
(d) retrieving isotactic polystyrene.

9. Isotactic polystyrene having an isotactic index of at least about 93% obtainable by the process of claim 8.

10. Isotactic polystyrene according to claim 9, wherein the polydispersity index Ip is from about 1 to about 15.

11. Isotactic polystyrene according to any one of claims 9 and 10, wherein the polystyrene molecular-weight distribution is essentially monomodal.

12. Isotactic polystyrene according to any one of claims 9 to 11, wherein the number average molecular weight Mn is from about 30,000 to about 100,000 g/mol.

13. Isotactic polystyrene according to any one of claims 9 to 12, wherein the isotactic index is at least about 95%.
